# EUROPEAN PATENT APPLICATION

(11) **EP 0 950 658 A1**
(43) Date of publication of application: **20.10.1999**
(21) Application number: 99302812.5
(22) Date of filing: 12.04.1999
(51) Int. Cl.: C07D 241/08, A61K 31/495

(54) **2-Pipirazinone-1-acetic acid dihydrochloride derivative used to inhibit platelet aggregation**

(30) Priority: 13.04.1998 JP 10142298
(71) Applicant: Takeda Chemical Industries, Ltd., Osaka 541-0045 (JP)
(72) Inventor: Mitsudera, Hiroyuki, Toyonaka, Osaka 560-0043 (JP)
(74) Representative: Lewin, John Harvey

(57) **Abstract**

The present invention is to provide a compound of (S)-4-(4-guanidinobenzoylamino)acetyl -3-[3-(4-guanidinobenzoylamino)]propyl-2-oxopiperazine-1-acetic acid dihydrochloride (I) which has potent and durable platelet aggregation inhibiting action, which is highly safe and which has superior physical and chemical properties, and production and use thereof. (I) is also used to inhibit cell adhesion.

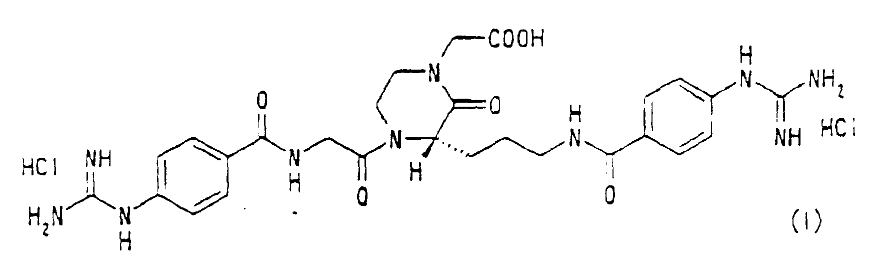

## Description

### Field of the Invention

This invention relates to a novel 2-pipirazinone-1-acetic acid derivative having a platelet aggregation inhibitory action, whose undesirable side effects such as bleeding, etc. are slight, and to a pharmaceutical composition for inhibiting cell-adhesion comprising said derivative as an effective component.

### Background of the Invention

As the factors participating in adhesion to extra-cellular matrix of animal cells, there have been known fibronection, vitronection, osteopontin, collagen, thrombospondin, fibrinogen, von willebrand factor, etc. These proteins include tripeptide -Arg-Gly-Asp- as a cell-adhesion recognition site. This tripeptide is recognized by at least one protein belonging to integrin receptors, which are heterodimeric proteins consisting of subunits combined with membranes [Science, 238, 491 (1987)].

Structurally related integrin receptors, which recognize the amino acid sequence: -Arg-Gly-Asp-, are known to express at the extra-cellular surface, glycoprotein of platelets, endothelial cells, leucocyte, lymphocyte, monocyte and granulocyte. Compounds having the amino acid sequence: -Arg-Gly-Asp- are competitively bound to the site to be bound with intracellular adhesive factors to thereby inhibit the binding of intracellular adhesive factors. As such substances for inhibiting intracellular adhesion, there has been known, for example, H-Gly-Arg-Gly-Asp-Ser-Pro-OH.

When blood vessels are injured, platelets are activated with, for example, endothelial collagens, etc., which causes binding of fibrinogen to platelets, i.e. platelet aggregation, to form thrombi. The interaction between platelets and fibrinogen takes place through GP IIb/IIIa, this being an important characteristic feature of platelet aggregation. Cell adhesion-inhibiting substances can inhibit platelet aggregation due to substances causing platelet aggregation such as thrombin, epinephrine, ADP, collagen, etc.

Besides, cell-adhesion inhibiting substances are expected to be useful as drugs for suppression of metastasis of tumor cells (inhibition of fixed adhesion at the site where the tumor cells have migrated).

Linear or cyclic peptides containing the amino acid sequence, -Arg-Gly-Asp- (RGD) have been known as cell-adhesion inhibiting substances [Journal of Biological Chemistry (J. Biol. Chem.), 262, 17294 (1987) and Japanese published unexamined patent application No. 174797/1990, among others].

And, non-peptide compounds having an anti-thrombotic action are disclosed in Japanese published unexamined patent application No. 264068/1992 and EPA 505868, in which compounds having 4- to 7-membered cyclic alkyleneimino such as pyrrolidine ring, etc. and compounds having piperidine ring, etc. are respectively described. Further, compounds having piperidinone ring, which have cell-adhesion inhibiting action, are disclosed in EPA 529858. And, such drugs as aspirin, heparin, ticlopidine, etc. are known to show undesirable side effects such as prolongation of bleeding time. As known platelet aggregation inhibiting substances which are slight in the action of prolonging bleeding time, cyclic peptide derivatives are described in Japanese publication of translations of International patent application No. 509551/1994.

On the other hand, piperazinone derivatives, which have superior cell-adhesion inhibiting action and platelet aggregation inhibiting action (main effect), whose action of prolonging bleeding time (side effect) is slight, and whose durability of the cell-adhesion inhibiting action and oral absorbability are improved, are disclosed in WO96/33982.

### Object of the Invention

As described above, various cell-adhesion inhibiting substances, which have platelet aggregation suppressing action and whose undesirable side effects such as bleeding, etc. are slight, is known. However, such compounds, which have a more potent main effect, whose side effect is reduced, which shows longer durability of the action, which can be orally administered, and whose physical and chemical properties such as stability, hygroscopicity, etc. are improved, have been sought for.

### Summary of the Invention

The present inventors diligently made extensive studies and, as a result, they succeeded for the first time in synthesizing a compound of (S)-4-(4-guanidinobenzoyl-amino)acetyl-3-[3-(4-guanidinobenzoylamino)]propyl-2-oxopiperazine-1-acetic acid dihydrochloride represented by the formula (I): 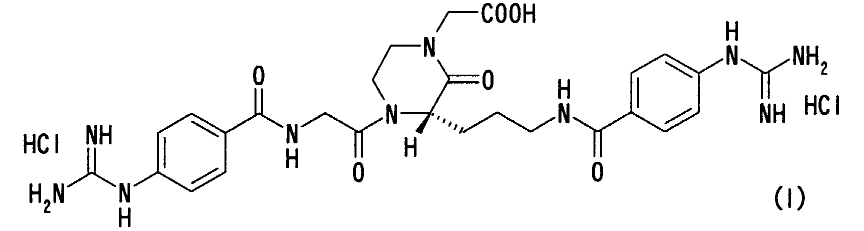 (hereinbelow, referred to as Compound (I)), and found that said compound has potent and durable platelet aggregation inhibiting action, based on its characteristic feature in the chemical structure, it is highly safe (that is, its undesirable side effects such as prolonging bleeding time, etc. are slight), and it has superior physical and chemical properties (that is, it is highly stable, it is easily crystallized, its hygroscopicity is improved, etc.). The inventors made further investigations based on this finding, and developed the present invention.

More specifically, the present invention relates to
(1) Compound (I);
(2) a pharmaceutical composition comprising the compound as described in the above (1);
(3) a pharmaceutical composition for inhibiting cell-adhesion, comprising the compound as described in the above (1);
(4) the composition according to the above (2), which is for the prevention or treatment of angina pectoris.;
(5) the composition according to the above (2), which is for the prevention or treatment of unstable angina;
(6) the composition according to the above (2), which is for the prevention or treatment of ischemic complication, reobstruction or restenosis after percutaneous transluminal coronary angioplasty or coronary thrombolytic therapy;
(7) a method for producing (S)-4-(4-guanidinobenzoylamino)acetyl-3-[3-(4-guanidinobenzoylamino)]-propyl-2-oxopiperazine-1-acetic acid of the formula: 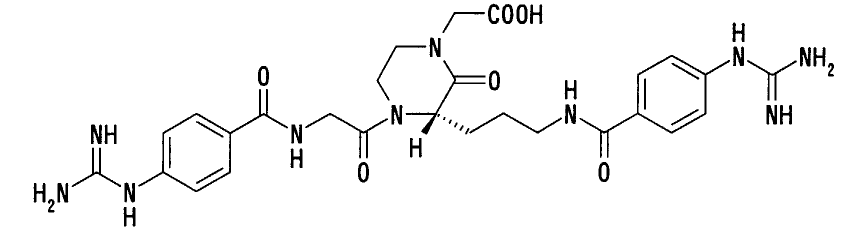 or a salt thereof, which comprises reacting 4-guanidinobenzoic acid of the formula (II): 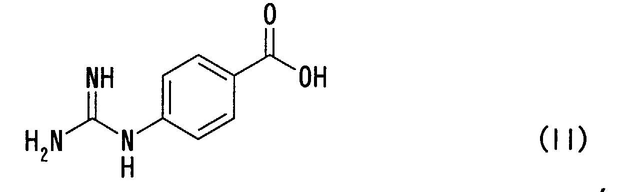 its reactive derivative or a salt thereof with (S)-4-glycyl-3-(3-aminopropyl)-2-oxopiperazine-1-acetic acid of the formula (III): 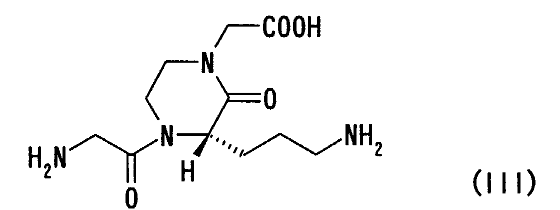 or a salt thereof in a mixture of acetonitrile and water;
(8) a method for producing the compound as described in the above (1), which comprises adding concentrated hydrochloric acid to a solution containing (S)-4-(4-guanidinobenzoylamino)acetyl-3-[3-(4-guanidinobenzoylamino)]propyl-2-oxopiperazine-1-acetic acid of the formula: 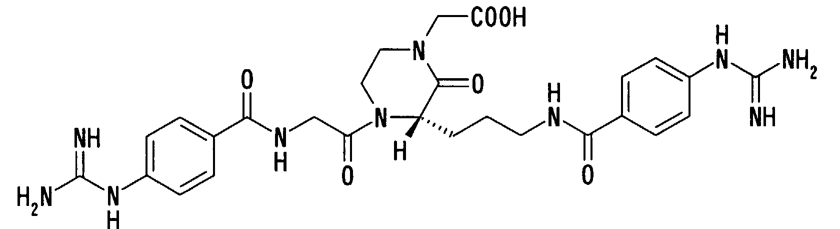 or a salt thereof to adjust pH of the solution to about 1-2;
(9) a method for producing crystals of the compound as described in the above (1), which comprises adding ethanol to a solution containing the compound as described in the above (1);
(10) a crystal of the compound as described in the above (1);
(11) a method for producing the compound as described in the above (1), which comprises reacting 4-guanidinobenzoic acid of the formula: 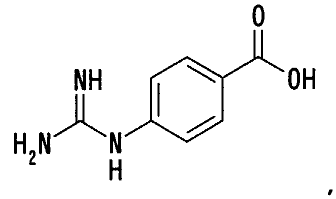 its reactive derivative or a salt thereof with (S)-4-glycyl-3-(3-aminopropyl)-2-oxopiperazine-1-acetic acid of the formula: 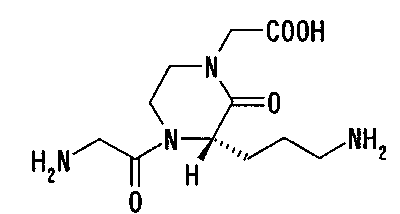 or a salt thereof and adding concentrated hydrochloric acid to a solution containing the obtained (S)-4-(4-guanidinobenzoylamino)acetyl-3-[3-(4-guanidinobenzoylamino)]-propyl-2-oxopiperazine-1-acetic acid of the formula: 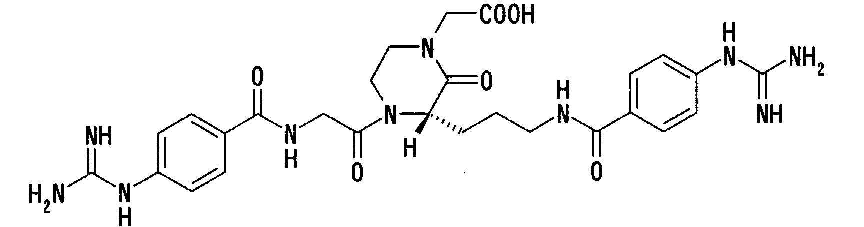 or a salt thereof to adjust pH of the solution to about 1-2; etc.

Compound (I) of the present invention has more potent main effects (cell-adhesion inhibiting action and platelet aggregation inhibiting action), its side effect (that is, prolonging bleeding time, etc.) is reduced, it shows longer durability of main effect and it is highly safe. Therefore, Compound (I) is superior in view of its pharmacological action. In addition, Compound (I) is superior in view of its physical and chemical properties as shown below.
(1) It is stable.
(2) Its hygroscopicity is low.
(3) It is easily crystallized.
(4) When its crystals are obtained from its solution, strict pH control is not necessary.
(5) The content of Cl in the crystals are constant.
(6) Its crystal form hardly changes.

Moreover, Compound (I) is superior in view of its production method as shown below.
(1) Its mass production is possible with simple operation.
(2) It can be produced with highly safe production methods.
(3) Disposal of waste solution or substance can be simplified.
(4) It can be produced by a production method where impurity does not mix with a final product.
(5) It can be produced by shortened steps and in good yield.
(6) It can be produced by a production method which does not need a reagent such as trifluoroacetic acid (TFA), etc., the treatment of which is not easy in view of stimulus, volatility, etc.
(7) It can be produced by a production method which does not need the treatment such as elution with ion exchange resin, etc. and concentration, said treatment requiring a long time.
(8) It can be produced under mild conditions (around neutral, room temperature, etc.).
(9) It can be produced by a production method where the amount of catalyst or starting materials is reduced.
(10) It can be produced by a production method where the amount of impurity production is small and a final product is colorless.

Compound (I) of the present invention can be produced by, for example, the following method or an analogous method thereto.

That is, Compound (I) can be produced by reacting 4-guanidinobenzoic acid of the formula (II): 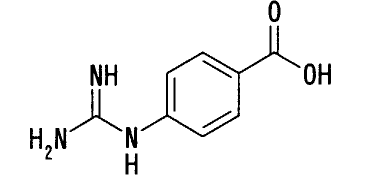 [Compound (II)], its reactive derivative or a salt thereof with (S)-4-glycyl-3-(3-aminopropyl)-2-oxopiperazine-1-acetic acid of the formula (III): 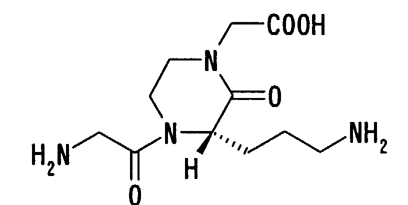 [Compound (III)] or a salt thereof to produce (S)-4-(4-guanidinobenzoylamino)acetyl-3-[3-(4-guanidinobenzoylamino)]propyl-2-oxopiperazine-1-acetic acid of the formula (I'): 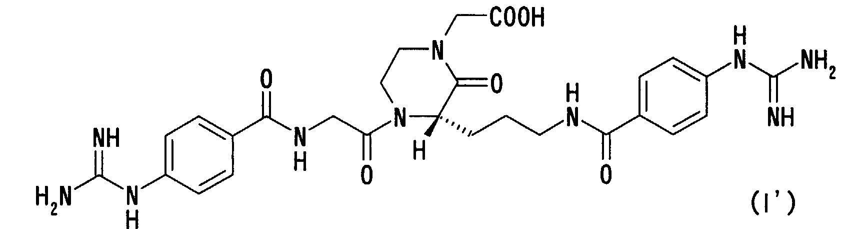 [Compound (I')] or a salt thereof, and then adding concentrated hydrochloric acid to a solution containing Compound (I') to adjust the pH of the solution to about 1-2.

Examples of salts of Compound (II) or Compound (III) include pharmaceutically acceptable salts, for example, inorganic acid salt such as hydrochloride, hydrobromide, sulfate, nitrate, phosphate, etc.; organic acid salt such as acetate, tartarate, citrate, fumarate, maleate, toluenesulfonate, methanesulfonate, etc.; metal salt such as sodium salt, potassium salt, calcium salt, aluminum salt, etc.; salt with base such as triethylamine salt, guanidine salt, ammonium salt, hydrazine salt, quinine salt, cinchonine salt, etc.; etc.

Examples of the reactive derivative of Compound (II) include, for example, a compound of the formula: 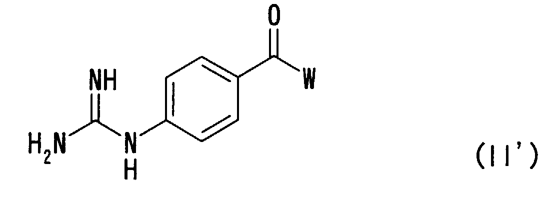 wherein W is halogen [Compound (II')] or acid anhydrides, azides, active esters [esters with alcohol (for example, pentachlorophenol, 2,4,5-trichlorophenol, 2,4-dinitrophenol, cyanomethanol, paranitrophenol, N-hydroxy-5-norbornene-2,3-dicarboxyimide, N-hydroxysuccinimide, N-hydroxyphthalimide, N-hydroxybenztriazole)], etc. corresponding to Compound (II). The active esters and in particular esters with N-hydroxy-5-norbornene-2,3-dicarboxyimide are preferably employed.

The condensation reaction as a production method of the compound of the present invention can be carried out by an amide-linkage formation reaction in a conventional peptide synthesis, for example, the method using active ester, mixed acid anhydride or acid chloride.

For example, the condensation reaction between Compounds (II) and (III) can be conducted by subjecting Compound (II) to condensation with a phenol such as 2,4,5-trichlorophenol, pentachlorophenol, 2-nitrophenol, 4-nitrophenol, etc. or an N-hydroxy compound such as N-hydroxysuccinimide, N-hydroxy-5-norbornene-endo-2,3-dicarboxyimide, 1-hydroxybenztriazole, N-hydroxypiperidine, etc. in the presence of a condensation reagent such as dicyclohexylcarbodiimide, etc. to convert into an active esters thereof, followed by condensation. Alternatively, Compound (II) is allowed to react with isobutyl chloroformate to give a mixed acid anhydride, which is then subjected to condensation.

Further, the condensation reaction between Compound (II) or its reactive derivative and Compound (III) can be also performed by using singly a peptide-formation reagent such as dicyclohexylcarbodiimide, N,N-carbonyldiimidazole, diphenylphosphoryl azide, diethyl cyanophosphate, etc.

In said condensation reaction, about 2-5 moles of Compound (II) are usually used per mole of Compound (III) and organic base (e.g., triethylamine, N-methylpiperidine, 4-N,N-dimethylaminopyridine, etc.) or inorganic base (sodium hydrogen carbonate, sodium carbonate, potassium carbonate, etc.) are preferably added to promote the reaction. The reaction temperature ranges usually from -20 to +50°C, preferably from 0°C to about +30°C, more preferably around room temperature. The reaction time varies depending on kinds of the solvents (including mixing ratio in the case of a mixed solvent), the reaction temperature, etc. and ranges usually from 1 minute to 72 hours, preferably from about 15 minutes to about 5 hours. Examples of the solvents usually employed, include, for example, a mixed solvent of acetonitrile and water, and the ratio of acetonitrile and water in said mixed solvent is preferably acetonitrile : water = 1 : 0.1-5, more preferably acetonitrile : water = 1 : 0.2-3, and in particular preferably acetonitrile : water = 1 : 0.3-2.

Compound (I') obtained by the above condensation reaction can be converted into Compound (I) by adding concentrated hydrochloric acid to a solution containing Compound (I') (preferably, a solution where Compound (I') is dissolved in an aqueous solvent) to adjust pH of the solution to about 1-2 (preferably 1.5).

While Compound (I) obtained by the above production method can be isolated from the reaction mixture by a conventional separation and purification means such as extraction, concentration, neutralization, filtration, recrystallization, column chromatography, thin layer chromatography, etc., it is preferable to precipitate crystals of Compound (I) by adding 5-10 times volume of ethanol to a solution containing Compound (I) (preferably, a solution where Compound (I) is dissolved in an aqueous solvent) and, if necessary, stirring and cooling the solution.

The starting Compounds (II) and (III) in the present invention are per se known compounds, or can be produced in a manner analogous to per se known methods. While Compound (III) can be produced by a method analogous to per se known methods, it can also be produced by, for example, the following reaction scheme:

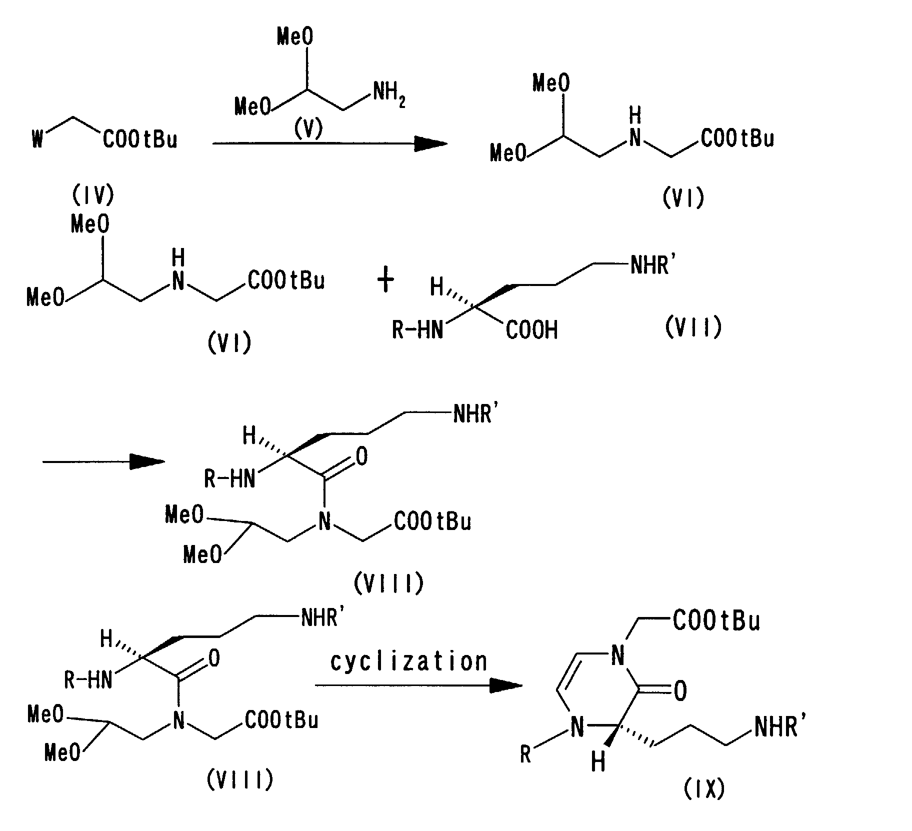

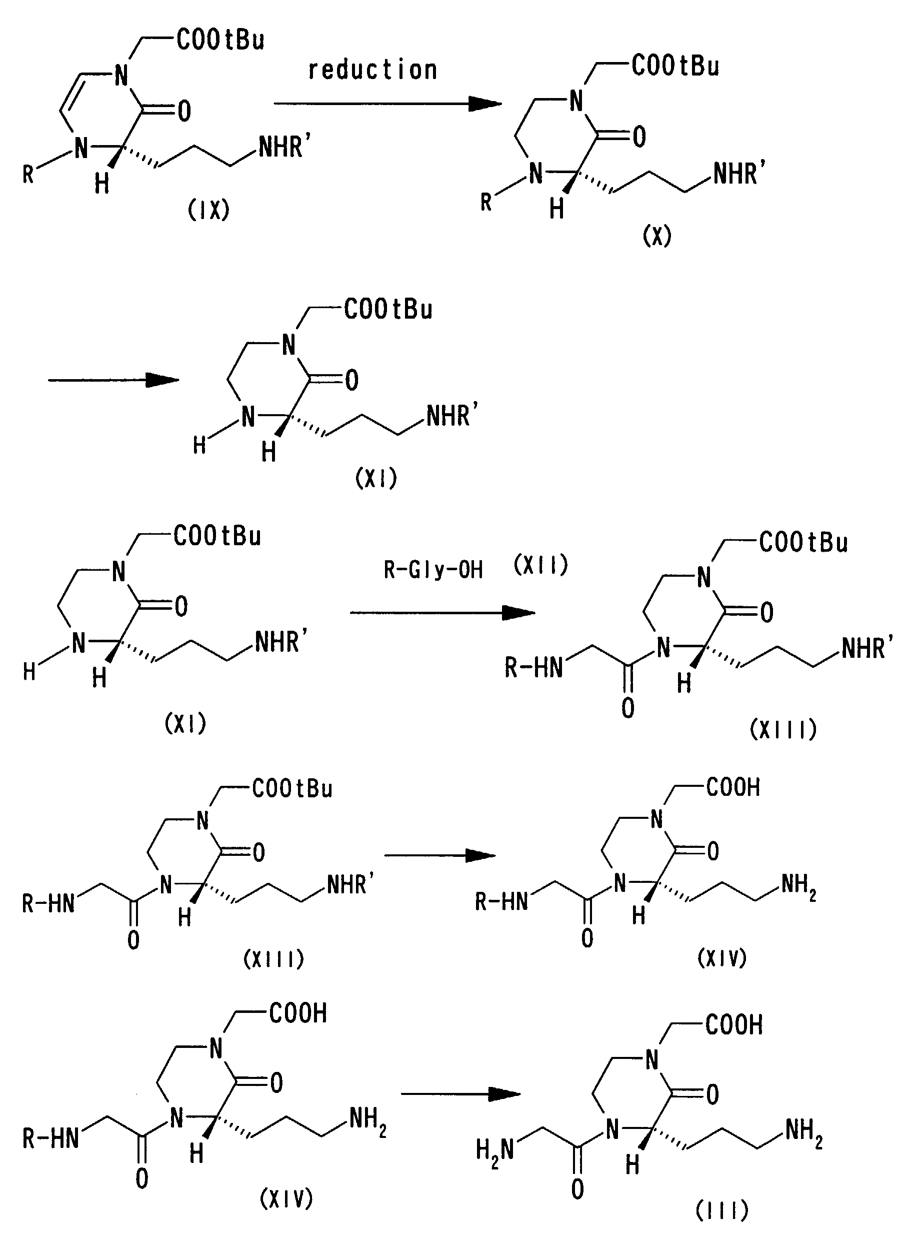

In the above formulas, R and R' are same or different protective groups for an amino group, preferably benzyloxycarbonyl group or tert-butoxycarbonyl group, and more preferably, R and R' are independently benzyloxycarbonyl group or tert-butoxycarbonyl group; and W is a halogen (Cl, Br, F, etc., preferably Cl).

The production method of Compound (III) shown by the above reaction scheme is explained in further detail. The reaction for obtaining Compound (VI) by reacting Compound (IV) with Compound (V) is a conventional alkylation of amino group. More specifically, Compound (IV) is reacted with Compound (V) in the presence of base (e.g., inorganic base such as sodium carbonate, potassium carbonate, potassium hydrogen carbonate, cesium fluoride, etc., or organic base such as triethylamine, pyridine, 4-N,N-dimethylaminopyridine, etc., etc.), usually at the temperature ranging from 0 to 100°C, preferably around room temperature, for about 15 minutes to about 5 hours. Examples of the reaction solvent include organic solvent such as acetonitrile, N,N-dimethylformamide, tetrahydrofuran, toluene, methylenechloride, etc.

The subsequent reaction for producing Compound (VIII) by subjecting Compound (VI) to condensation reaction with Compound (VII) is a conventional peptide-linkage formation reaction, which can be conducted under substantially the same reaction conditions as the condensation reaction of Compound (II) with Compound (III).

Cyclization of Compound (VIII) into Compound (IX) is a cyclization reaction with an acid catalyst. Examples of the catalyst include, for example, p-toluenesulfonic acid, camphorsulfonic acid, methanesulfonic acid, etc. Compound (IX) can be produced by conducting the reaction usually in a solvent such as toluene, benzene, ethyl acetate, 1,2-dichloroethane, etc. at a temperature ranging from 0 to 100°C, preferably from 30 to 80°C.

The subsequent reaction for reducing Compound (IX) to Compound (X) can be conducted by catalytic reduction using, as a catalyst, for example, metal such as platinum, palladium, Raney-nickel, etc. or a mixture thereof with an optional carrier, or a reduction using, for example, a metallic hydride such as sodium borohydride, etc. The reaction is usually carried out in an organic solvent (e.g., methanol, ethanol, dioxane, ethyl acetate, etc.). The reaction temperature preferably ranges from -20 to 100°C. This reaction can be conducted under normal pressure or under elevated pressure. When R is a benzyloxycarbonyl group, the reaction for removing the protective group of R proceeds simultaneously to obtain Compound (XI) by catalytic reduction.

The reactions for removing protective groups in (X) to (XI), in (XIII) to (XIV) and in (XIV) to (III) are conventional reactions for removing protective groups of amino groups. When R or R' is benzyloxycarbonyl group, the protective group can be removed by catalytic reduction using, as a catalyst, metal such as platinum, palladium, Raney-nickel, etc. or a mixture thereof with an optional carrier. And, when R or R' is tert-butoxycarbonyl group, the protective group can be easily removed by the use of an acid such as trifluoroacetic acid, hydrogen chloride, etc. in an organic solvent such as methanol, ethanol, ethyl acetate, dioxane, etc.

The condensation reaction of Compound (XI) with Compound (XII) is a conventional peptide-linkage formation reaction, which can be conducted under substantially the same reaction conditions as the condensation reaction of Compound (II) with Compound (III).

In the above-mentioned methods for producing Compound (I) and its intermediates, compounds to be employed for the reactions may, unless undesirable effects are brought about, be in the form of a salt with, for example, an inorganic acid such as hydrochloride, hydrobromide, sulfate, nitrate, phosphate, etc., an organic acid such as acetate, tartarate, citrate, fumarate, maleate, toluenesulfonate, methanesulfonate, etc.; a metal salt such as sodium salt, potassium salt, aluminum salt, etc.; a salt with a base such as triethylamine salt, guanidine salt, ammonium salt, hydrazine salt, quinine salt, etc.

Compound (I) (including its hydrates) is low in toxicity and used safely, which inhibits both the binding of fibrinogen, fibronection, and von willebrand factor to fibrinogen receptors of blood platelets (Glycoprotein IIb/IIIa) and the binding thereof and other adhesive proteins, such as vitronection, collagen and laminin, to the corresponding receptors on the surface of various types of cells.

Hence, the present compound exerts influence on cell-cell and cell-matrix interactions. It prevents, in particular, the development of thrombus and can be used for the treatment or prevention of diseases such as angina pectoris, unstable angina, acute myocardial infarction, Kawasaki disease, acute and chronic cardiac insufficiency, transient ischemic attack (TIA), cerebral apoplexy, cerebral ischemic disturbance in acute phase of cerebral thrombosis, dissecting aneurysm of the aorta, cerebral vasospasm after subarachnoid hemorrhage, acute or chronic renal diseases (for example, acute or chronic renal diseases due to overagglutination such as snake venom and immunopathy), chronic and acute glomerulonephritis, diabetic nephritis and nerve disturbance, nephrotic syndrome, liver diseases, pulmonary embolism, bronchial asthma, pulmonary edema, adult respiratory distress syndrome (ARDS), arteriosclerotic obliteration, peripheral arterial obstruction, deep vein thrombopsis, vibration disease, peripheral arterial obstruction complicated with diabates mellitus, thrombotic thrombocytopenic purpura (TTP), disseminated intravascular coagulation (DIC), sepsis, surgical or infective shock, postoperative and postdelivery trauma, premature separation of placenta, incompatible blood transfusion, systemic lupus erythematosus, Raynaud's disease, inflammation, arteriosclerosis, hemolytic uremic syndrome, symmetric peripheral necrosis, bedsore, hemorrhoids, etc. in mammals including human (for example, mouse, rat, guinea pig, dog, rabbit, human, etc.). And, Compound (I) of the present invention can be used for preventing thrombosis due to cardiopulmonary bypass surgical operation, surgical operation pump oxygenator, atrial fibrillation or fracture of hip joint, prosthetic valve replacement, artificial blood vessel and organs, etc., or preventing thrombocytopenia during artificial dialysis, and further for secondary prophylaxis of myocardial infarction. The preventing of thrombocytopenia during artificial dialysis also means preventing coagulation or non-washable blood in shunt of extracorporeal dialysis.

Further, Compound (I) of the present invention can be used for coronary thrombolytic therapy (e.g. enhancing the action of thrombolytic agent such as tissue plasminogen activator (TPA) and preventing reobstruction), for preventing reobstruction and restenosis of coronary arteries after PTCA (percutaneous transluminal coronary angioplasty) or stent-indwelling and atherectomy, for preventing reobstruction and restenosis after surgical operation for coronary artery bypass, for preventing ischemic complication (e.g. myocardial infarction, death) after PTCA or coronary thrombolytic therapy, and, besides Compound (I) of the present invention can inhibit cancer metastasis and be used as an antitumor agent.

And, in case where the present Compound (I) is used together with a drug whose pharmacological action is same as or different from that of the compound of this invention, two or more kinds of drugs may be incorporated into one and the same pharmaceutical preparation, or these components can be incorporated into one and the same pharmaceutical preparation (e.g. powdery preparation and injection) at the time of administration.

Further, pharmaceutical preparations independently formulated may be administered to one and the same subject simultaneously or at an optional time lag.

Pharmaceutical compositions containing Compound (I) of the present invention (including its hydrates and salts) can be administered orally in the form of, for example, tablets, lacquered tablets, sugar-coated tablets, hard and soft gelatin capsules, solutions, emulsions or suspensions, or rectally in the form of suppositories, or as spray. However, administration can also be performed non-orally, for example, in the form of injectable solutions.

Pharmaceutical preparations of the above-mentioned forms can be formulated by respectively conventional methods using, when necessary, adequate excipients.

To prepare tablets, lacquered tablets, sugar-coated tablets and gelatin capsules, an active compound can be mixed with pharmaceutically inert inorganic or organic excipients. Typical examples of such excipients, which can be used for tablets, sugar-coated tablets and gelatin capsules, include lactose, corn starch or derivatives thereof, talc, and stearic acid or salts thereof. Examples of suitable excipients for soft gelatin capsules are vegetable oil, wax, fat, and, semisolid or liquid polyol. However, no excipients whatever are necessary with soft gelatin capsules when physical and chemical properties of the active compound are appropriate.

Examples of suitable excipients for the preparation of solutions and syrupy preparations are water, polyol, sucrose, invert sugar and glucose. Suitable examples for injectable solution are water, alcohol, polyol, glycerol and vegetable oil.

Suitable examples for suppositories are natural or hardened oil, wax, fat and semiliquid or liquid polyol. The pharmaceutical compositions can additionally contain a preservative, a solubilizer, a stabilizer, a wetting agent, an emulsifier, a sweetener, a colorant, a flavoring, a salt to alter the osmotic pressure, a buffer, a coating agent or an antioxidant.

The dosage of the active compound for controlling or preventing the diseases referred to hereinbefore can vary within a wide range and can, of course, be adjusted to suit the individual circumstances in each particular case. While the dosage varies with the subject diseases, symptoms, subject patients and administration routes, when administered orally to a patient of unstable angina, or, ischemic complication or reobstruction of coronary or restenosis of coronary after PTCA or coronary thrombolytic therapy, a dose of about 1 to 500 mg, preferably about 10 to 200 mg of the compound (I), per day for an adult (60 kg), divided into one to three times, is appropriate. When administered non-orally to a patient of transient ischemic attack (TIA), unstable angina, or, ischemic complication or reobstruction of coronary or restenosis of coronary after PTCA or coronary thrombolytic therapy, a dose of about 0.05 to 50 mg, preferably about 1 to 20 mg/kg of the compound (I), per day to an adult (60 kg), divided into one to three times, is appropriate.

### Best Mode for Carrying out the Invention

The present invention is hereinafter described in more detail by means of the following Reference Examples and Working Example which are not to be construed as limitative.

### Examples

### Reference Example 1

### Production of tert-butyl N-(2,2-dimethoxyethyl)glycinate [BMG]

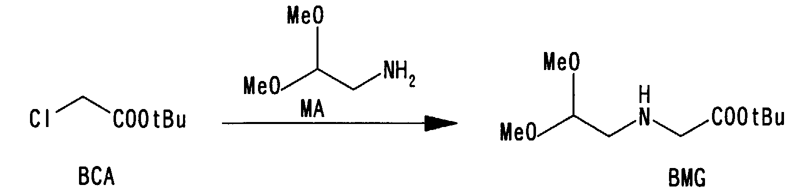

To acetonitrile 667ml was added 2,2-dimethoxyethylamine [MA] 105.1g (1.0mol), and then powdery potassium carbonate 139.6g while stirring. To the mixture was added tert-butyl chloroacetate [BCA] 75.3g (0.5mol), and the mixture was stirred at 23°C for 2 hours. To the mixture was added BCA 75.3g (0.5mol), and the mixture was stirred at 30°C for 16 hours. Insoluble materials were filtered off, and the residue was washed with acetonitrile 83ml. The filtrate was collected, and the solvent was evaporated under reduced pressure. The residue was evaporated under reduced pressure to give 120.6g (55%, 107-115/0.5mmHg) of tert-butyl N-(2,2-dimethoxyethyl)glycinate [BMG].

### Reference Example 2

### Production of tert-butyl [N-(2,2-dimethoxy)-N-(N-benzyloxycarbonyl-N-t-butoxycarbonyl)ornithinyl]-glycinate [BMOG]

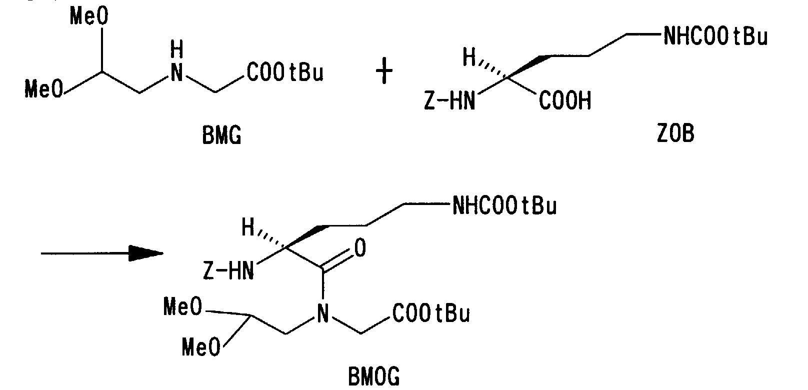

wherein Z is a benzyloxycarbonyl group.

To acetonitrile 3.0L were added (N-benzyloxycarbonyl-N-tert-butoxycarbonyl)ornithine (Z-L-Orn(Boc)-OH [ZOB]) 378.7g(1.034mol) and BMG 226.7g (1.034mol), and then aqueous carbodiimide (WSC) 256.8g (1.348mol), divided into a few times, while stirring at room temperature. The mixture was stirred at the same temperature for 1 hour, and the reaction solution was poured into 5% potassium hydrogen sulfate solution 3.0L. The mixture was extracted with ethyl acetate 3.2L, and the extract was washed with saturated sodium bicarbonate solution 2.0L and saturated brine 2.0L, dried with anhydrous magnesium sulfate 190g, filtered and washed with ethyl acetate 100ml. The filtrate was collected, and the solvent was evaporated under reduced pressure to give BMOG 581.3g (102.4%).

### Reference Example 3

### Production of benzyl (S)-2-(3-tert-butoxycarbonylaminopropyl)-4-tert-butoxycarbonylmethyl-3-oxo-3,4-dihydro-2H-pyrazine-1-carboxylate [BBPC]

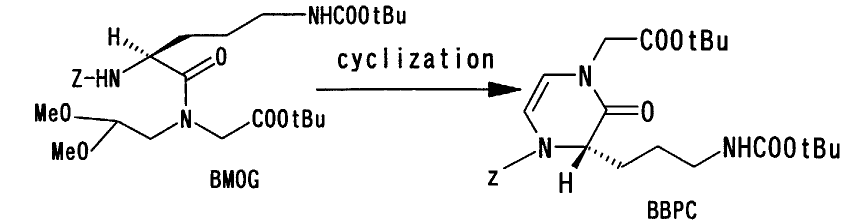

In toluene 9.0L were dissolved BMOG 446.9g (0.787mol) and p-toluenesulfonic acid (p-TsOH·H₂O) 15.0g(0.0787mol), and the mixture was stirred at 70°C for 2 hours. To the mixture was added p-TsOH·H₂O 15.0g (0.0787mol), and the mixture was stirred at 70°C for 1 hour. The mixture was concentrated to half volume under reduced pressure to remove produced methanol. To the residue was added toluene 5.0L, and the mixture was stirred at 70°C for 1 hour. The reaction solution was poured into saturated sodium bicarbonate solution 4.4L, and the mixture was separated. The organic layer was washed with saturated brine 4.4L, dried with anhydrous magnesium sulfate 300g, filtered and washed with toluene 200ml. The filtrate was collected, and the solvent was evaporated under reduced pressure. The residue was purified with silica gel column chromatography (eluent; AcOEt/n-Hexane=4/6-5/5) to give BBPC 254.9g (64.3%).

### Reference Example 4

### Production of tert-butyl (S)-3-(3-tert-butoxycarbonylaminopropyl)-2-oxopiperazine-1-acetate [BOPA]

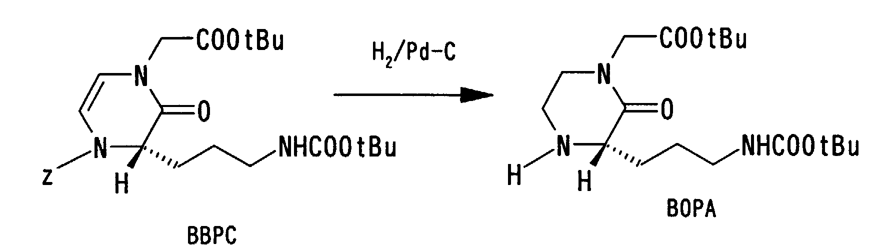

In ethyl acetate 8.0L was dissolved BBPC 270g (0.585mol), and to the mixture was added 10% Pd-C 107.5g. The mixture was stirred under hydrogen atmosphere for 1 hour. After completion of the reaction, the catalyst was filtered off. The filtrate was combined and the solvent of the mixture was evaporated under reduced pressure. The residue was dissolved in methanol 520ml, and to the residue was added oxalic acid dihydrates 73.8g (0.585mol). The precipitated crystals were filtered, which was washed with ethyl acetate 100ml to give BOPA (oxalate) 254.9g (64.3%).

### Reference Example 5

### Production of tert-butyl (S)-4-benzyloxycarbonylaminoacetyl-3-(3-tert-butoxycarbonylaminopropyl)-2-oxopiperazine-1-acetate [BGPA]

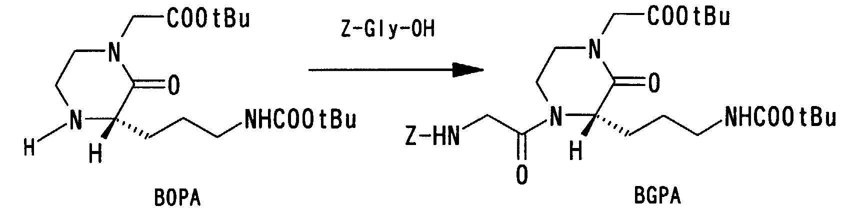

In ethyl acetate 2.0L was suspended BOPA (oxalate) 200g (0.43mol), and to the suspension was added saturated sodium bicarbonate solution 2.0L. The mixture was vigorously stirred for 1 hour, and the ethyl acetate layer was separated. The aqueous layer was extracted with ethyl acetate 500ml. The organic layer was washed with water (1.0L) and concentrated under reduced pressure. The residue and N-benzyloxycarbonylglycine (Z-Gly-OH) 99.6g (0.476mol) were dissolved in acetonitrile 2.0L, and to the mixture was added WSC 103g (0.562mol). The mixture was stirred at room temperature for 3 hours and concentrated under reduced pressure. The obtained oil was dissolved in ethyl acetate 2.0L, and the mixture was washed with 5% potassium hydrogen sulfate solution 2.0L and saturated sodium bicarbonate solution 2.0L, and evaporated under reduced pressure. The residue was dissolved in ethyl acetate (AcOEt) (100ml), and to the mixture was added isopropylether (IPE) 700ml to precipitate crystals, to which was added IPE 300ml. The mixture was stirred under ice-cooling for 1 hours, and precipitated crystals were filtered, which were dried at 40°C for 6 hours under reduced pressure to give BGPA 223g (91.5%).
m.p. 113-114°C

### Reference Example 6

### Production of (S)-4-(benzyloxycarbonylaminoacetyl)-3-(3-aminopropyl)-2-oxopiperazine-1-acetic acid [ZAPA]

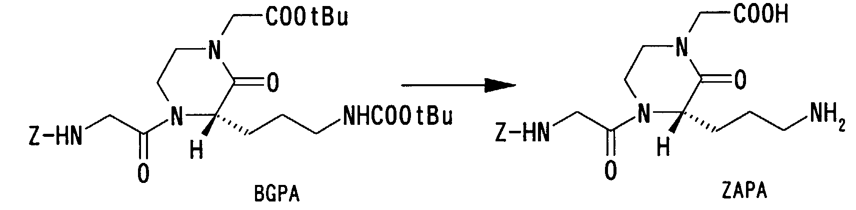

In concentrated hydrochloric acid 1.0L was dissolved BGPA 1.0kg (1.8mol), and the mixture was stirred at room temperature for 1-2 hours. To the mixture was added water 3.0L, and neutralized (pH6-7) with 6N-NaOH (about 2.0L) while keeping inner temperature 15-30°C to give ZAPA as a solution.

### Reference Example 7

### Production of (S)-4-glycyl-3-(3-aminopropyl)-2-oxopiperazine-1-acetic acid [GAPA]

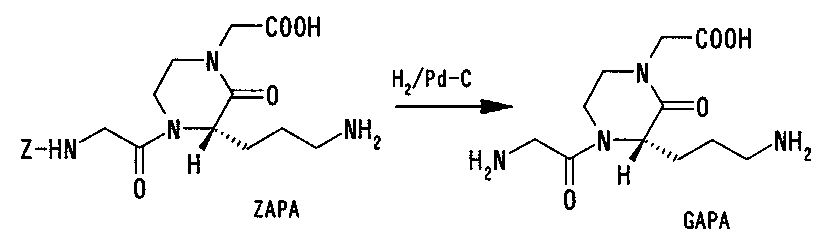

To the solution of ZAPA obtained in Reference Example 6 was added water 3.0L to make the total amount of the solution 10L. To the solution was added 5% Pd-C 200g, and the mixture was stirred under hydrogen atmosphere at room temperature for 3 to 4 hours. After the reaction was completed, the catalyst was filtered off, and the filtrate was washed with 50% acetonitrile 5.0L to give GAPA as a solution (total amount: about 15L).

### Reference Example 8

### Production of N-(4-guanidinobenzoyloxy)-5-norbornene-2,3-dicarboxyimide [GBNB]

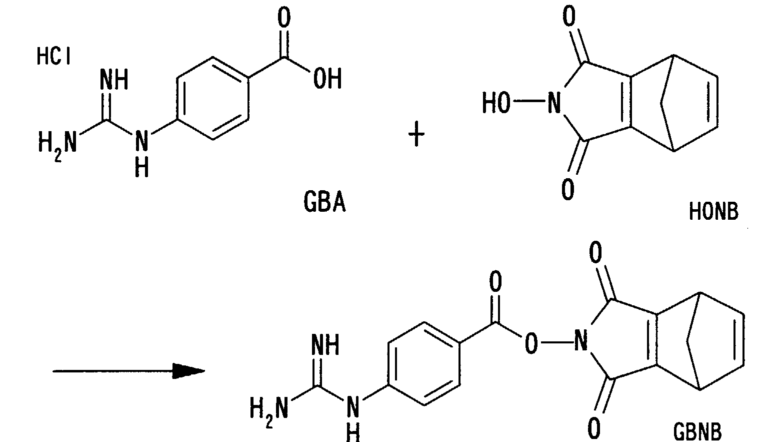

In DMF 3.0L, were dissolved 4-guanidinobenzoic acid hydrochloride 439g (2.04mol) and N-hydroxy-5-norbornene-2,3-dicarboxyimide (HONB) 434g (2.42mol), and to the mixture was added under ice-cooling (at 10°C or less) DCC 500g (2.42mol). The mixture was allowed to stand at room temperature for 2 hours. The precipitate was filtered off, and the filtrate was concentrated under reduced pressure. The residue was dissolved in methanol 1.5L, and to the solution was added IPE (about 4.0L) until the solution became a white suspension, which was stirred at room temperature for 2 hours. Precipitated crystals were filtered to give 667g (75%) of GBNB.

### Working Example 1

### Production of (S)-4-(4-guanidinobenzoylamino)acetyl-3-[3-(4-guanidinobenzoylamino)]propyl-2-oxopiperazine-1-acetic acid dihydrochloride

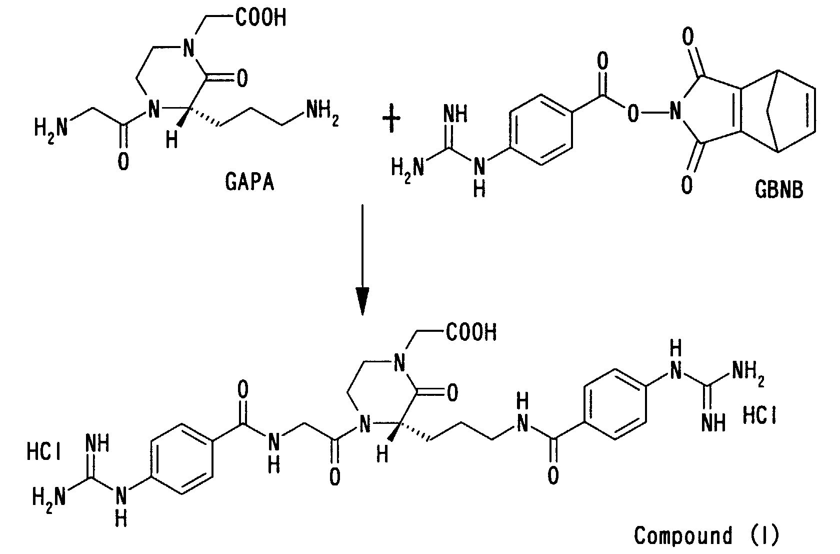

To the solution of GAPA obtained in Reference Example 7, were added acetonitrile 7.0L, water 6.6L and sodium hydrogen carbonate 448g (5.33mol), and then the active esters (GBNB) 1607g (4.27mol) obtained in Reference Example 8. The mixture was stirred at room temperature for 4 hours, and the reaction solution was adjusted to pH3 with 2N-HCl and extracted with ethyl acetate (30L×3). The aqueous layer was concentrated to about 10L under reduced pressure, and to the residue was added water 10L. The mixture was adjusted to pH5.0 with sodium bicarbonate, loaded on the resin (SP-207, 30L-column), washed with pure water 150L and eluted with 0.003N-HCl/5% acetonitrile 250L. The desired fractions (about 200L) were collected and concentrated to about 10L under reduced pressure. The concentrate was adjusted to pH1.5 with concentrated hydrochloric acid (about 117ml) and then concentrated to 3L. To the residue was added ethanol 24L, and the mixture was stirred at room temperature for 19 hours and then under ice-cooling for 2 hours. The precipitated crystals were filtered, washed with 89% ethanol 900ml, air-dried overnight, and vacuum-dried at 50°C for 9 hours to give crude crystals of Compound (I) 819g. The crude crystals of Compound (I) was dissolved in water 2.05L, and to the solution was added active charcoal 16.5g. The mixture was stirred at room temperature for 30 minutes and filtered. The filtrate was passed through 0.2µ membrane filter, to which was added ethanol 20.5L. The mixture was stirred at room temperature for 6 hours, and then under ice-cooling for 2 hours. The precipitated crystals were filtered, which were washed with 89% ethanol (1.0L), vacuum-dried at 50°C for 9 hours, allowed to stand at RH100% overnight, vacuum-dried at 50°C for 9 hours, and allowed to stand at RH60-70% for about 3 days to give purified crystals of Compound (I) (dihydrochloride) 753g (Yield: 61.7%, BGPA standard) containing 5% water (about 2 moles).
m.p.: 245-251.5°C

| Elemental Analysis for C₂₇H₃₄N₁₀O₆ · 2HCl · 1.5H₂O | | | | |
|---|---|---|---|---|
| Calcd. | C, 46.69; | H, 5.66; | H, 20.17; | Cl,10.21 |
| Found | C, 46.15; | H, 5.62; | H, 19.94; | Cl,10.65 |

### Effect of the Invention

Compound (I) of the present invention has potent, continuous and highly safe (that is, minimal undesirable side effects such as prolonging bleeding time, etc.) platelet aggregation inhibiting action, and also has superior physical and chemical properties such as high stability, easy crystallization, improved hygroscopicity, etc. Therefore, it is advantageously used as cell-adhesion inhibitor, anti-thrombotic agent, etc.

## Claims

1. (S)-4-(4-guanidinobenzoylamino)acetyl-3-[3-(4-guanidinobenzoylamino)]propyl-2-oxopiperazine-1-acetic acid dihydrochloride.

2. A pharmaceutical composition comprising the compound as claimed in claim 1.

3. A pharmaceutical composition for inhibiting cell-adhesion, comprising the compound as claimed in claim 1.

4. The composition according to claim 2, which is for the prevention or treatment of angina pectoris.

5. The composition according to claim 2, which is for the prevention or treatment of unstable angina.

6. The composition according to claim 2, which is for the prevention or treatment of ischemic complication, reobstruction or restenosis after percutaneous transluminal coronary angioplasty or coronary thrombolytic therapy.

7. A method for producing (S)-4-(4-guanidinobenzoylamino)acetyl-3-[3-(4-guanidinobenzoylamino)]propyl-2-oxopiperazine-1-acetic acid or a salt thereof, which comprises reacting 4-guanidinobenzoic acid, its reactive derivative or a salt thereof with (S)-4-glycyl-3-(3-aminopropyl)-2-oxopiperazine-1-acetic acid or a salt thereof in a mixture of acetonitrile and water.

8. A method for producing the compound as claimed in claim 1, which comprises adding concentrated hydrochloric acid to a solution containing (S)-4-(4-guanidinobenzoylamino)acetyl-3-[3-(4-guanidinobenzoylamino)]propyl-2-oxopiperazine-1-acetic acid or a salt thereof to adjust pH of the solution to about 1-2.

9. A method for producing crystals of the compound as claimed in claim 1, which comprises adding ethanol to a solution containing the compound as claimed in claim 1.

10. A crystal of the compound as claimed in claim 1.

11. A method for producing the compound as claimed in claim 1, which comprises reacting 4-guanidinobenzoic acid, its reactive derivative or a salt thereof with (S)-4-glycyl-3-(3-aminopropyl)-2-oxopiperazine-1-acetic acid or a salt thereof and adding concentrated hydrochloric acid to a solution containing the obtained (S)-4-(4-guanidinobenzoylamino)acetyl-3-[3-(4-guanidinobenzoylamino)]propyl-2-oxopiperazine-1-acetic acid or a salt thereof to adjust pH of the solution to about 1-2.

12. Use of the compound as claimed in claim 1 for the manufacture of a medicament for inhibiting cell-adhesion.

13. Use of the compound as claimed in claim 1 for the manufacture of a medicament for the prevention or treatment of angina pectoris.

14. Use of the compound as claimed in claim 1 for the manufacture of a medicament for the prevention or treatment of unstable angina.

15. Use of the compound as claimed in claim 1 for the manufacture of a medicament for the prevention or treatment of ischemic complication, reobstruction or restenosis after percutaneous transluminal coronary angioplasty or coronary thrombolytic therapy.
